# NEW EUROPEAN PATENT SPECIFICATION

(11) **EP 0 913 127 B2**
(45) Date of publication and mention of the opposition decision: **21.06.2006**
(45) Mention of the grant of the patent: 06.09.2000
(21) Application number: 98115299.4
(22) Date of filing: 14.08.1998
(51) Int. Cl.: A61B 18/20

(54) **Device for elimination of hairs and/or atrophying hair follicles**
Vorrichtung zum Entfernen von Haaren und/oder zum Atrophieren der Haarfollikel
Dispositif pour l'épilation et/ou pour atrophier les follicules pileux

(30) Priority: 26.09.1997 IT BO970579
(43) Date of publication of application: 06.05.1999
(73) Proprietor: Etoile S.n.c., 20060 Robbiano di Mediglia (IT); EL.EN. S.p.A., 50100 Firenze (IT)
(72) Inventor: Lazzari, Ambrogio, 20060 Robbiano di Mediglia (IT); Clementi, Gabriele, 50139 Firenze (IT)
(74) Representative: Gustorf, Gerhard

(56) References cited:
- EP-A- 0 438 353
- WO-A-91/06406
- US-A- 4 905 670
- US-A- 5 065 515
- US-A- 5 562 842
- US-A- 5 632 741
- EUROPEAN NORM EN 60601-2-22
- Review "DENTAL CADMOS"
- Review "LASERS"
- Data sheet "ETOILE"

## Description

The present invention relates to a device for elimination of hairs and/or atrophying hair follicles,

At present, various devices are known for obtaining elimination of superfluous and/or unwanted hair for aesthetic purposes or for skin transplants, as are various devices for obtaining atrophying of the hair follicles, in order to inhibit or delay re-growth of the hairs.

Some of these devices substantially comprise an operative console, combined with a terminal unit which emits electromagnetic and/or light energy, for example laser rays, which is connected by means of cables to the said operative console, in which the said terminal unit can be positioned manually by the operator in the vicinity of the epidermal surface, in order to be able to irradiate the latter diffusely and locally.

Documents US 5 632 741 and US 5 653 706 disclose devices with features according to the preamble of present claim 1.

In order to use the said devices with diffuse irradiation, the operator must first of all apply and spread regularly on the area of epidermis to be treated a substance/composition which has high absorption of light at a particular frequency band (laser), and, subsequently, must bring the aforementioned irradiation unit into the vicinity of the area concerned, and illuminate it diffusely with the light which has the aforementioned specific frequency band (laser), in order to impart energy to the energy-absorbing substance/composition which is disposed on the epidermis, which in turn, by being heated and/or reacting, eliminates the hairs andlor atrophies the hair follicles.

These particular devices have a series of disadvantages.

A first disadvantage is derived from the fact that the area to be treated is subjected to diffuse radiation of laser rays, which damages not only the hairs/hair follicles, but also the part of the epidermis which surrounds the said hairs/hair follicles.

A second disadvantage, which is correlated to the above-described problem, is derived from the fact that it is not possible to carry out intensive hair-removal treatment and/or atrophying of the hair follicles, because it is necessary to moderate the intensity (energy) of the irradiation rays, in order to limit the damage caused by the irradiation diffused on the skin.

A third disadvantage is derived from the fact that it is necessary to spread the energy-absorbing substance/composition on the skin very carefully and regularly, in order to prevent a so-called "leopard spot" treatment, in which some areas are excessively treated, whereas others are insufficiently treated.

The object of the present invention is to eliminate the above-described disadvantages.

The invention, which is characterised by the claims, solves the problem of creating a device for elimination of hairs and/or for atrophying of hair follicles.

The object of the present invention is thus a device as defined in claim 1.

The following results are obtained by means of use of a device of this type: the light ray which is emitted by the terminal unit reaches, and acts on, only the hairs and/or the hair follicles to be treated, without affecting the surrounding epidermis; and, additionally, the operator can position the terminal unit rapidly, without errors, and in rapid succession, in order to carry out the treatment on the individual hairs/hair follicles to be treated.

A first advantage which is obtained by means of the present invention consists substantially in that by carrying out treatment which is aimed at individual hairs/follicles, i.e. by avoiding diffuse irradiation of the epidermis, the latter is less damaged, since the cells of the epidermis which surround the hair/hair follicles are not irradiated, and thus are not damaged.

A second advantage which is obtained by means of the present invention consists in that, by observing the point of incidence of the guide ray on the epidermis, firstly with the naked eye, and subsequently on the enlarging screen, the operator can aim the terminal unit quickly, easily, safely, and with micrometric accuracy, thus guaranteeing perfect centring of the destructivelatrophying light ray which will then be emitted.

A third advantage of the present invention consists in that, after having carried out treatment, the operator can check whether it has been successful, and if considered necessary, can treat once again the hair/hair follicle already treated.

Further features and advantages of the present invention will become more apparent from the following detailed description of a preferred practical embodiment, provided purely by way of non-limiting example, in which there are described two embodiments of the unit, also provided purely by way of non-limiting example, with reference to the figures of the attached drawings in which:
Figure 1 is a schematic view of the device which is the subject of the present invention;
Figure 2 is a schematic view of a first embodiment of the terminal unit according to the present invention;
Figure 3 is a schematic view of a second embodiment of the terminal unit according to the present invention.

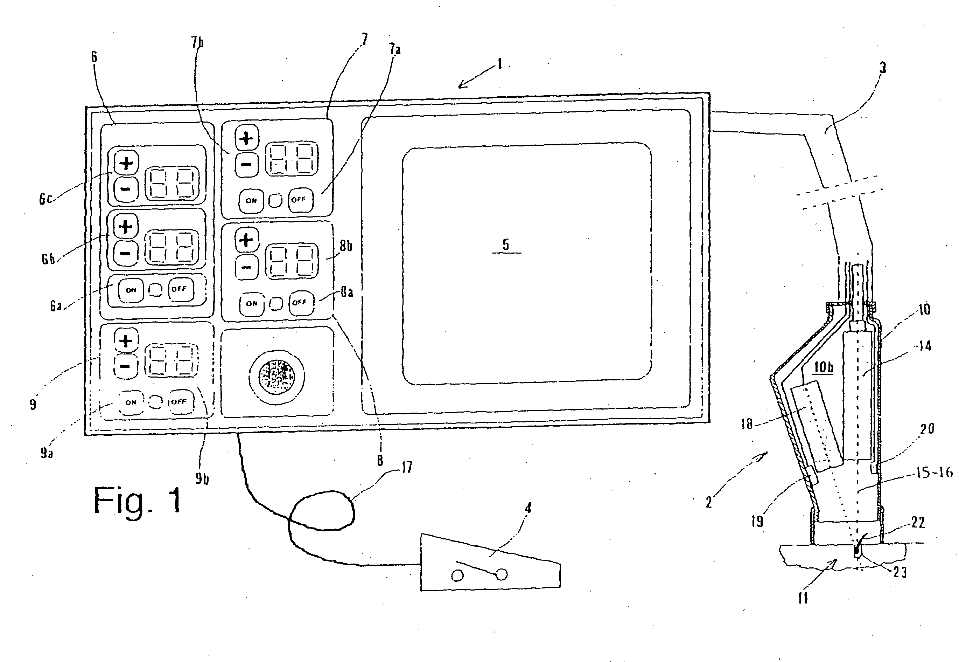

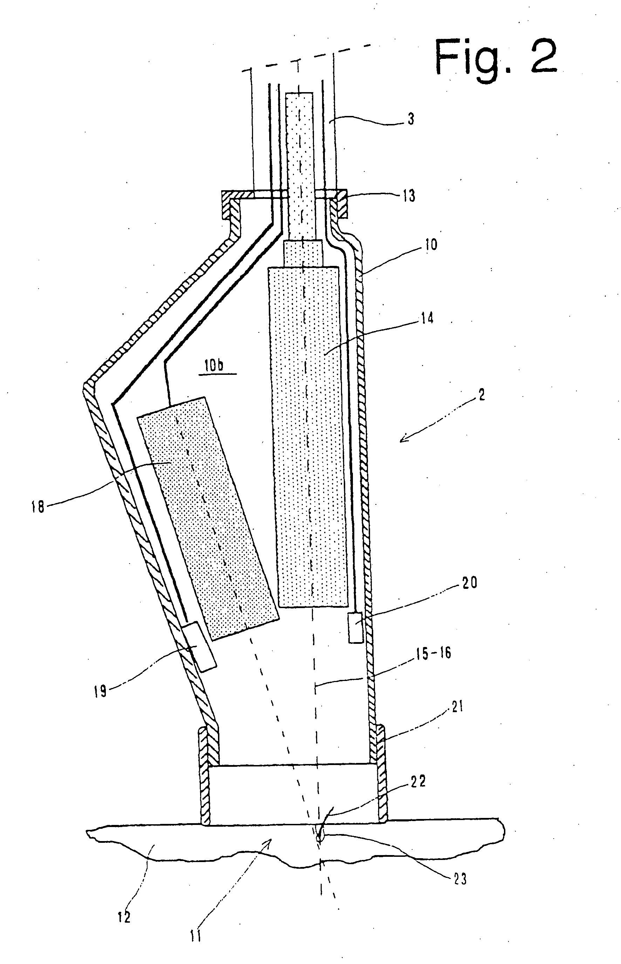

With reference to figure 1, the device according to the present invention comprises: an operative console which is indicated 1 as a whole; a terminal unit which is indicated 2 as a whole, which is separate from the said operative console 1, but is connected to the latterby means of cables 3; and a command pedal 4, which is also connected to the console 1 by means of a cable 17.

The operative console 1 comprises a monitor 5, and adjacent to the latter, a control panel which is divided into sectors 6, 7, 8 and 9, for control of the said device. The monitor 5 can also be designed such that it is separate from the control panel.

The sector indicated 6 is designed for control of a destructive/atrophying light ray, and in this example a laser ray, which is described in greater detail hereinafter, and comprises a switch 6a, a time regulator (timer) with an indicator 6b and an intensity regulator (energy) with an indicator 6c.

The sector indicated 7 is designed for control of a guide ray, which is described in greater detail hereinafter, and comprises a switch 7a, and an intensity regulator with an indicator 7b.

The sector indicated 8 is designed for control of a light source, which is described in greater detail hereinafter, and comprises a switch 8a, and an intensity regulator with an indicator 8b.

The sector indicated 9 is designed for control of a jet of air, which is described in greater detail hereinafter, and comprises a switch 9a, and a pneumatic flow regulator with an indicator 9b.

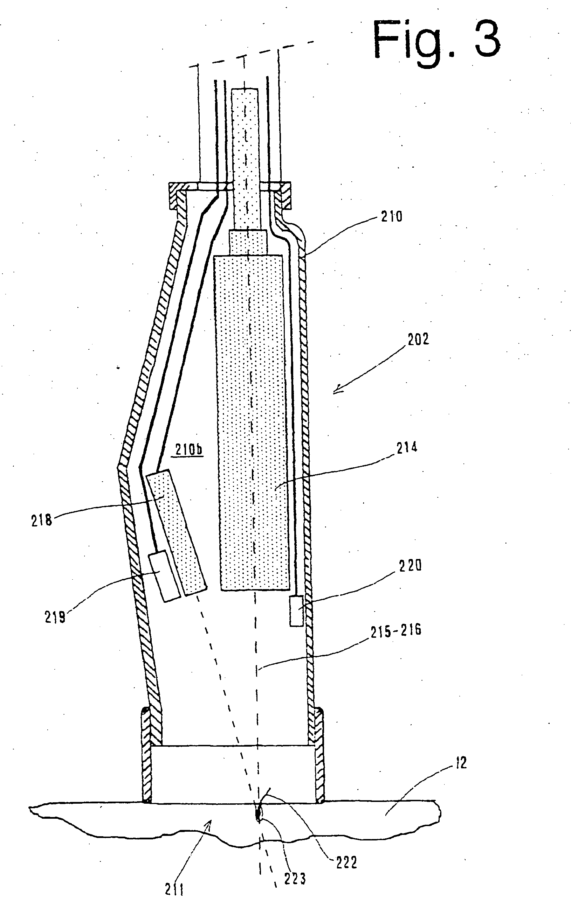

According to a first embodiment of the unit 2, see figure 2, the unit consists of a hollow body 10 with an elongate shape, which is designed to form an inner chamber 10b, which in its lower part communicates with the exterior by means of an aperture 11, the outer edge of which is designed to be laid against the epidermis 12 to be treated. The upper part of the said hollow body 10 has a cover 13 through which the cables 3 pass.

Inside the terminal unit 2, there are disposed four elements, and more specifically a light-focusing pointer 14, for example a laser-focusing pointer, which faces the said aperture 11, and can project a guide ray 15 with a small cross-section, and, on command, a destructive/atrophying light ray 16, which also has a small cross-section, which rays are uni-directional and co-axial to one another, directly towards the exterior through the said aperture 11; optical retrieval means, consisting of a micro-telecamera 18, which is connected to the monitor 5, faces the said aperture 11, and is designed to frame directly through the said aperture 11 the portion of the epidermis 12 reached by the said guide ray 15 and the immediate surrounding area, and then to reproduce in enlarged form on the monitor 5 the images retrieved; a light source 19, which is designed to light the epidermis 12 through the said aperture 11, when the epidermis is being abutted; and, finally, a nozzle 20 which is connected to known pneumatic means not illustrated or described, and, on command or continuously, can direct a puff of compressed air towards the said aperture 11, and thus against the said epidermal surface 12 when it is being abutted.

The guide ray 15 and the destructive/atrophying light ray 16 have the same transverse cross-section, or different transverse cross-sections which are selected in relation to the treatment to be carried out, and, again by way of non-limiting example, preferably a circular transverse cross-section with a diameter of between 0.05mm and 2mm.

On its end part, the aperture 11 has an end sleeve 21, which is secured in a detachable manner to the lower part of the unit 2, such that the sleeve can be replaced if required, for example for a different patient.

The command pedal 4 is a command push-button of a known type.

When the device is switched on, i.e. when it is ready for use, the light-focusing pointer 14 projects the guide ray 15 continuously through the aperture 11, the micro-telecamera 18 retrieves the images which appear in front of the aperture 11, the light source 19 illuminates whatever is in front of the aperture 11 , the nozzle 20 blows air through the said aperture, and the pedal 4 is enabled.

In this operative situation, when the push-button pedal 4 is pressed, by means of known electrical and/or electronic devices which are not described or illustrated here, the said guide ray 15 is de-activated, and there is activation of the destructive/atrophying light ray 16, the intensity of which is pre-determined by means of the regulator 6c, for a time which is pre-determined by means of the regulator 6b, in order then to re-project the guide ray 15.

After having regulated the device by means of the aforementioned sectors 6, 7, 8 and 9, the operator grasps the unit 2 with one hand, and places one foot on the pedal push-button 4, without pressing it.

Then, observing with the naked eye a hair 22 and/or a hair follicle 23 to be treated, the operator brings the unit 2 quickly towards the epidermal surface, maintaining the point of incidence of the guide ray 15 above and centred, relative to the hair/follicle 22/23 to be treated.

When the aperture 11 of the unit reaches the epidermal surface 12, by observing the monitor 5, the operator can see in a considerably enlarged form the epidermal surface reached by the guide ray 15, and if required, can carry out minor displacements of the unit in order to obtain perfect centring of the guide ray 15 relative to the said hair/follicle 22/23.

At this point, when the centring has been consolidated, by pressing the pedal 4, the operator emits the destructive/atrophying light ray 16, which, owing to its small transverse cross-section, reaches perfectly the hair/hair follicle 22/23 alone which was centred by the guide ray 15, thus preventing irradiation of the epidermis which surrounds the said hair/hair follicle 22/23.

The destructivelatrophying light ray 16 thus emitted destroys/atrophies the hair/hair follicle 22 /23, and the fumes which are generated by the micro-burning are immediately expelled by the continuous jet of air generated by the nozzle 20, thus permitting an immediate clear view on the monitor 5 of the epidermis 12 treated.

As well as carrying out a function of "washing" which is directed towards the area of epidermis treated, the said jet of air also carries out a function of alleviating the pain/discomfort suffered by the patient, by cooling quickly the micro-burnt area of epidermis 12. If required, instead of acting continuously, the said jet can also be enabled to blow only after the destructive/atrophying light ray 16 has been emitted.

At this point, by observing the monitor 5, the operator can determine whether the treatment has been successful, and if required, can press the pedal push-button 4 once again, and emit a further destructivelatrophying light ray 16 onto the hair/hair follicle 22/23 already treated.

When the operator considers that the treatment has been completed on a specific hair/hair follicle, the unit 2 must be moved to a new position, and in order to carry out this operation there are two alternatives, i. e. if the new hair/hair follicle is close to the one previously treated, the operator will move the terminal unit 2 without raising it from the epidermis, by watching the monitor 5; whereas if the new hair/hair follicle to be treated is far from the one previously treated, e.g. outside the field framed by the monitor 5, the operator will remove/raise the aperture 11 from the epidermis 12, in order to recommence the above-described process.

Figure 3 illustrates purely by way of non-limiting example a second practical embodiment of the unit, in this case indicated 202, which consists of a hollow body 210 with an elongate shape, which is designed to form an inner chamber 210b, which, on its lower part, communicates with the exterior by means of an aperture 211 which is designed to be laid against the epidermis 12 to be treated.

Inside the terminal unit 202 there are disposed four elements, i.e. a light-focusing pointer 214, which is similar to the one previously described, and can project a guide ray 215 with a small cross-section, and, on command, a destructive/atrophying light ray 216 which also has a small cross-section, which rays are co-axial to one another, towards the exterior, through the said aperture 211; a light source 219 which projects a beam of light through the said aperture 211 and thus against the epidermis 12 when it is abutted; a nozzle 220 which is connected to known pneumatic means not illustrated or described, and can direct a puff of compressed air on command or continuously towards the said aperture 211 and thus against the said epidermal surface 12 when it is abutted; and finally, an optical fibre 218, the free end of which, which is disposed inside the unit 202, on which the lens, not illustrated, is disposed, frames through the said aperture 211 the portion of epidermis 12 which faces it, and in particular the area of epidermis which the guide ray 215 meets, and the immediate surrounding area. At the other end of the same optical fibre 218 there is connected a telecamera, of a known type and not illustrated, which is disposed for example inside the operative console 1, which is connected to the monitor 5.

As in the preceding case, the operator will first of all bring the unit 202 quickly towards the hair/hair follicle 222/223 to be treated, observing with the naked eye the point of incidence of the guide ray 215 on the epidermis, and will then centre the guide ray 215 directly and micrometrically relative to the hair/hair follicle 222/223 to be treated, by viewing the monitor which reproduces the enlarged image of the epidermis disposed against the aperture 211, and finally, will emit the timed destructive/atrophying light ray 216, for removal of the said hair/atrophying of the hair follicle 222/223.

The present invention can be used to burn superfluous hairs, as well as to atrophy hair follicles, and in the latter case, the hairs can still be present, or can be extracted before the treatment.

In addition, the present invention can be used on a bare epidermis, i.e. an epidermis which is clean and untreated, or on a prepared epidermis, on which for example any substance/composition which can improve and/or facilitate and/or optimise and/or intensify the treatment has previously been applied.

Finally, again with reference to the present invention, it must be emphasized that the light of the destructive/atrophying light ray 16, 216, can be of different types, i.e. white, infra-red, polarised (laser), or of any other type.

The description of the device for elimination of hairs and/or atrophying of hair follicles is provided purely by way of non-limiting example, and it is thus apparent that there can be made to it all the modifications and variants suggested by practice and by its utilisation or use, still within the scope of the following claims.

## Claims

1. Device for elimination of hairs and/or for atrophying hair follicles, comprising an operative console and a mobile terminal unit connected to the said operative console, designed to be positioned in the vicinity of the epidermis, the said terminal unit (2) comprising a body. (10) which has an inner chamber (10b) which communicates with the exterior by means of an aperture (11), wherein inside the said chamber (10b) there is disposed a light-focusing pointer (14) which is designed to project on command a destructive/atrophying ray (16) having a small cross-section, said command being set off by a command means (4) for commanding emission of the destructive/atrophying light ray (16), said light ray being directed towards the exterior via the aperture (11) and towards the epidermis (12) when the epidermis (12) is brought against the said aperture (11);
**characterized in that** said light-focusing pointer (14) is also designed to project a first guide ray (15), which has a small cross-section and which is co-axial to the said second destructive/atrophying light ray (16) and directed towards the exterior via the aperture (11);
**in that** the said light-focusing pointer (14) faces the aperture (11) and projects said first guide ray (15) and said second destructive/atrophying light ray (16) directly towards the said aperture (11) and directly against the epidermis (12), if the latter is laid against the aperture itself (11);
**in that** inside the said chamber (10b) there are disposed optical retrieval means (18) which face the aperture (11) and are designed to retrieve directly through the said aperture (11) the images which appear in front of the aperture (11) itself and the image of the epidermis (12) reached by the said guide ray (15) and by the said destructive/atrophying light ray (16), if the said epidermis (12) is laid against the aperture (11),
**in that** the said optical retrieval means (18) are connected to a monitor (5) which can be seen by the operator, and which reproduces in enlarged form the images retrieved by the said optical retrieval means (18),
and **in that** means (6b) are provided for pre-determining the duration of the destructive/atrophying light ray (16; 216) projected from said light-focusing pointer (14; 150; 151; 214) on command after said first guide ray (15; 215).

2. Device according to claim 1, **characterised in that** the said destructive/atrophying light ray (16; 216) is a laser ray.

3. Device according to claim 1 or 2, **characterised in that** the said optical retrieval means (18; 218) comprise a micro-telecamera (18) which is connected to a monitor (5) which reproduces in an enlarged form the images retrieved.

4. Device according to any one of claims 1 to 3, **characterised in that** the said optical retrieval means (18; 218) comprise an optical fibre (218) the free end of which, which admits the image, is disposed inside the unit (202), and the other end of the said optical fibre (218) ends outside the said unit (202) and is connected to an external telecamera, which in turn is connected to the monitor (5) which reproduces in an enlarged form the images retrieved.

5. Device according to any one of the preceding claims, **characterised in that** the said command means (4) for the destructive/atrophying light ray comprise a push-button pedal (4).

6. Device according to any one of the preceding claims, **characterised in that** means (6c) are provided for regulation of the intensity and/or the energy of the destructive/atrophying light ray (16).

7. Device according to any one of the preceding claims, **characterised in that** in the interior (10b; 210b) of the said unit (2; 202) there is also provided a light source (19; 219) which can illuminate through the said aperture (11; 211) the epidermis (12), when the latter is disposed in the vicinity of the said aperture (11).

8. Device according to any one of the preceding claims, **characterised in that** in the interior (10b; 210b) of the said unit (2; 202) there is also provided a nozzle (20; 220) which can blow compressed air towards, and through, the said aperture (11; 211).

9. Device according to any one of the preceding claims, **characterised in that** the outer end part of the said aperture (11; 211) has a detachable/replaceable sleeve (21).

## Patentansprüche

1. Vorrichtung zum Entfernen von Haaren und/oder zum Atrophieren der Haarfollikel, umfassend ein Bedienpult und ein mit diesem verbundenes, mobiles Endgerät, das an die Epidermis angelegt werden kann, wobei das Endgerät (2) ein Gehäuse (10) mit einer inneren Kammer (10b) hat, die über eine Öffnung (11) nach außen offen ist und in der ein Licht fokussierendes Zielgerät (14) angebracht ist, über welches auf Befehl ein zerstörender/atrophierender Strahl (16) mit kleinem Querschnitt ausgesandt wird, wobei der Befehl ausgelöst wird durch ein Auslöseorgan (4) zur Steuerung der Abgabe des zerstörenden/atrophierenden Lichtstrahls (16) und der Lichtstrahl durch die Öffnung (11) nach außen und gegen die Epidermis (12) gerichtet wird, wenn diese zur Anlage an die Öffnung (11) gebracht worden ist, **dadurch gekennzeichnet, daß** das Licht fokussierende Zielgerät (14) auch zur Abgabe eines ersten Führungsstrahls (15) ausgelegt ist, der einen kleinen Querschnitt hat, koaxial zu dem zweiten, zerstörenden/atrophierenden Lichtstrahl (16) verläuft und durch die Öffnung (11) nach außen gerichtet ist,
daß das Licht fokussierende Zielgerät (14) der Öffnung (11) gegenüberliegt und den ersten Führungsstrahl (15) sowie den zweiten, zerstörenden/atrophierenden Lichtstrahl (16) direkt auf die Öffnung (11) und direkt gegen die Epidermis (12) richtet, wenn diese an der Öffnung (11) anliegt,
daß in der Kammer (10b) optische Aufspürmittel (18) angeordnet sind, die der Öffnung (11) gegenüberliegen und so ausgelegt sind, daß sie direkt durch die Öffnung (11) hindurch die Bilder, die sich vor der Öffnung (11) befinden, sowie das Bild der Epidermis (12) wiederfinden, die von dem Führungsstrahl (15) und dem zerstörenden/atrophierenden Lichtstrahl (16) erfaßt werden, wenn die Epidermis (12) an der Öffnung (11) anliegt,
daß die optischen Aufspürmittel (18) mit einem von der Bedienungsperson einsehbaren Monitor (5) verbunden sind, der die von den optischen Aufspürmitteln (18) erfaßten Bilder in vergrößerter Form darstellt,
und daß Mittel (6b) zur Voreinstellung der Dauer des zerstörenden/aptrophierenden Lichtstrahls (16, 216) vorgesehen sind, der von dem Licht fokussierenden Zielgerät (14; 150, 151; 214) auf Befehl im Anschluß an den ersten Führungsstrahl (15; 215) ausgesandt wird.

2. Vorrichtung nach Anspruch 1, **dadurch gekennzeichnet, daß** der zerstörende/atrophierende Lichtstrahl (16; 216) ein Laserstrahl ist.

3. Vorrichtung nach Anspruch 1 oder 2, **dadurch gekennzeichnet, daß** die optischen Aufspürmittel (18; 218) eine Mikrotelekamera (18) aufweisen, die mit einem Monitor (5) verbunden ist, welcher die erfassten Bilder in vergrößerter Form wiedergibt.

4. Vorrichtung nach einem der Ansprüche 1 bis 3, **dadurch gekennzeichnet, daß** die optischen Aufspürmittel (18; 218) eine Lichtleitfaser (218) aufweisen, deren freies Ende, welches das Bild aufnimmt, innerhalb der Einheit (202) liegt, während das andere Ende der Lichtleitfaser (218) außerhalb der Einheit (202) endet und mit einer äußeren Telekamera verbunden ist, welche ihrerseits mit dem Monitor (5) verbunden ist, der die erfassten Bilder in vergrößerter Form wiedergibt.

5. Vorrichtung nach einem der vorhergehenden Ansprüche, **dadurch gekennzeichnet, daß** das Auslöseorgan (4) für den zerstörenden/atrophierenden Lichtstrahl ein Drucktastenpedal (4) hat.

6. Vorrichtung nach einem der vorhergehenden Ansprüche, **dadurch gekennzeichnet, daß** Mittel (6c) zur Einstellung der Intensität und/oder Energie des zerstörenden/atrophierenden Lichtstrahls (16) vorgesehen sind.

7. Vorrichtung nach einem der vorhergehenden Ansprüche, **dadurch gekennzeichnet, daß** in dem Innenraum (10b; 210b) der Einheit (2; 202) eine Lichtquelle (19; 219) angeordnet ist, welche durch die Öffnung (11; 211) die Epidermis (12) beleuchten kann, wenn sich diese in der Nähe der Öffnung (11) befindet.

8. Vorrichtung nach einem der vorhergehenden Ansprüche, **dadurch gekennzeichnet, daß** sich im Innenraum (10b; 210b) der Einheit (2; 202) eine Düse (20; 220) befindet, die Druckluft gegen und durch die Öffnung (11; 211) bläst.

9. Vorrichtung nach einem der vorhergehenden Ansprüche, **dadurch gekennzeichnet, daß** am äußeren Teil der Öffnung (11; 211) eine abnehmbare/auswechselbare Hülse (21) angebracht ist.

## Revendications

1. Dispositif pour éliminer les cheveux et/ou pour atrophier les follicules de cheveux, comprenant une console fonctionnelle et un terminal mobile connecté à ladite console fonctionnelle, conçu pour être positionné au voisinage de l'épiderme, ledit terminal (2) comprenant un corps (10) qui possède une chambre intérieure (10b) qui communique avec l'extérieur au moyen d'une ouverture (11), dans lequel à l'intérieur de ladite chambre (10b) est disposé un pointeur à focalisation de lumière (14) qui est conçu pour projeter, selon une commande, un rayon destructeur/atrophiant (16) présentent une petite section, ladite commande étant mise à l'arrêt par un moyen de commande (4) pour commander l'émission du rayon lumineux destructeur/atrophiant (16), ledit rayon lumineux étant dirigé vers l'extérieur à travers l'ouverture (11) et vers l'épiderme (12) lorsque l'épiderme (12) est amené contre ladite ouverture (11),
**caractérisé en ce que** ledit pointeur à focalisation de lumière (14) est également conçu pour projeter un premier rayon de guidage (15) présentant une petite section, qui est coaxial avec le second rayon lumineux destructeur/atrophiant (16) et dirigé vers l'extérieur à travers l'ouverture (11);
**en ce que** ledit pointeur à focalisation de lumière (14) fait face à l'ouverture (11) et projette le premier rayon de guidage (15) et le second rayon lumineux destructeur/atrophiant (16) directement vers ladite ouverture (11) et directement contre l'épiderme (12), si ce dernier est tenu contre l'ouverture elle-même (11);
**en ce que** à l'intérieur de ladite chambre (10b) sont disposés des moyens de récupération optique (18) qui font face à l'ouverture (11) et qui sont conçus pour récupérer directement à travers l'ouverture (11) les images qui apparaissent en face de ladite ouverture (11), et l'image de l'épiderme (12) atteint par ledit rayon de guidage (15) et par ledit rayon lumineux destructeur/atrophiant (16) si ledit épiderme (12) est maintenu contre ladite ouverture (11);
**en ce que** lesdites moyens de récupération optique (18) sont connectés à un écran de contrôle (5) qui peut être vu par l'opérateur, et qui reproduit sous forme agrandie les images récupérées par lesdites moyens de récupération optique (18);
et **en ce que** des moyens (6b) sont prévus pour pré-régler la durée du rayon lumineux destructeur/atrophiant (16; 216) émis selon une commande par le pointeur à focalisation de lumière (14; 150, 151; 214) après le premier rayon de guidage (15; 215).

2. Dispositif selon la revendication 1, **caractérisé en ce que** ledit rayon lumineux destructeur/atrophiant (16; 216) est un rayon laser.

3. Dispositif selon la revendication 1 ou 2, **caractérisé en ce que** lesdits moyens de récupération optique (18; 218) comprennent une micro télécaméra (18) qui est connectée à un écran de contrôle (5) qui reproduit sous forme élargie les images récupérées.

4. Dispositif selon l'une quelconque des revendications 1 à 3, **caractérisé en ce que** lesdits moyens de récupération optique (18; 218) comprennent une fibre optique (218) dont l'extrémité libre, qui reçoit l'image, est disposée à l'intérieur du terminal (202), et l'autre extrémité de ladite fibre optique (218) se termine à l'extérieur dudit terminal (202) et est connectée à une télécaméra externe, qui à son tour est connectée à l'écran de contrôle (5) qui reproduit sous forme élargie les images récupérées.

5. Dispositif selon l'une quelconque des revendications précédentes, **caractérisé en ce que** ledit moyen de commande (4) pour le rayon lumineux destructeur/atrophiant comprend une pédale pousse-bouton (4).

6. Dispositif selon l'une quelconque des revendications précédentes, **caractérisé en ce que** des moyens (6c) sont prévus pour réguler l'intensité et/ou l'énergie du rayon lumineux destructeur/atrophiant (16).

7. Dispositif selon l'une quelconque des revendications précédentes, **caractérisé en ce que** dans l'intérieur (10b; 210b) dudit terminal (2; 202) on a également prévu une source de lumière (19; 219) qui peut éclairer à travers ladite ouverture (11; 211) l'épiderme (12), lorsque ce dernier est disposé au voisinage de ladite ouverture (11).

8. Dispositif selon l'une quelconque des revendications précédentes, **caractérisé en ce que** dans l'intérieur (10b; 210b) dudit terminal (2; 202) on a également prévu une buse (20; 220) qui peut souffler de l'air comprimé vers, et à travers, ladite ouverture (11; 211).

9. Dispositif selon l'une quelconque des revendications précédentes, **caractérisé en ce que** la
